(19) Europäisches Patentamt / European Patent Office / Office européen des brevets

(11) **EP 2 982 981 B1**

(12) **EUROPEAN PATENT SPECIFICATION**

(45) Date of publication and mention
of the grant of the patent:
**28.12.2016 Bulletin 2016/52**

(51) Int Cl.:
*G01N 33/543* [(2006.01)]      *H01L 21/768* [(2006.01)]
*G01N 27/327* [(2006.01)]      *G01N 27/414* [(2006.01)]

(21) Application number: **15177452.8**

(22) Date of filing: **20.07.2015**

(54) **SEMICONDUCTOR BIOSENSOR AND CONTROL METHOD THEREOF**

HALBLEITER-BIOSENSOR UND STEUERUNGSVERFAHREN DAFÜR

BIOCAPTEUR À SEMI-CONDUCTEUR ET SON PROCÉDÉ DE COMMANDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.08.2014 JP 2014169886**

(43) Date of publication of application:
**10.02.2016 Bulletin 2016/06**

(73) Proprietor: **Watanabe, Hiroshi
Hsinchu City, Taiwan (TW)**

(72) Inventors:
• **WATANABE, Hiroshi
Hsinchu City
Taiwan (R.O.C.) (TW)**
• **LEE, Zhe-An
Hsinchu City
Taiwan (R.O.C.) (TW)**
• **KURACHI, Ikuo
Hachio ji-shi
Tokyo (JP)**

(74) Representative: **Louis Pöhlau Lohrentz
Patentanwälte
Postfach 30 55
90014 Nürnberg (DE)**

(56) References cited:
**US-B2- 8 697 565**

• **KAZUTO KOIKE ET AL: "A potentiometric
immunosensor based on a ZnO field-effect
transistor", JAPANESE JOURNAL OF APPLIED
PHYSICS, vol. 53, no. 5S1, 14 April 2014
(2014-04-14), pages 05FF01-05FF04,
XP55233622, Tokyo ISSN: 0021-4922, DOI:
10.7567/JJAP.53.05FF04**

## Description

### 1. Field of the Invention

The present invention relates to a semiconductor biosensor which is used for healthcare chip and a control method of the semiconductor biosensor.

### 2. Description of the Related Art

In recent years, it has become significant to suppress the drastic increase of medical expense since the society is aging in developed countries. It may be possible to early find diseases and thereby reduce the medical expense if we can inspect and detect little amount of chemical substance contained in a sample with high precision. It is expected that such an inspect method can be achieved by utilizing microelectronics technologies. (See K. Koike, et al., Jpn. J. Appl. Phys., vol. 53, 05FF04 (2014).). US 8,697,565 B2 discloses an apparatus including a substrate having a metallization layer and a dielectric layer extending over the metallization layer; an external contact pads and a sensor contact pad extending through the dielectric layer; a conductive cap layer over the sensor contact pad; and external contacts directly overlying the external contact pad. However, US 8,697,565 B2 does not disclose the central reaction unit of the present application.

However, the inspection equipment with high precision is not only expensive but also large in size. Thus, the inspection can only be carried out in large hospitals or medical facilities. This makes the inspection expensive, and the inspection period is often more than several days.

In other words, small inspection equipment with high precision may enable small-scale medical institutes to perform cheap inspections.

The inspection period shall be substantially reduced with simplified process of inspection, then substantially reducing the cost and improving the convenience of the inspection. Therefore, in order to cut the cost of the inspection, it is necessary to substantially reduce the size of inspection equipment while keeping the precision by utilizing the combined technologies of semiconductor micro-devices and biosensors.

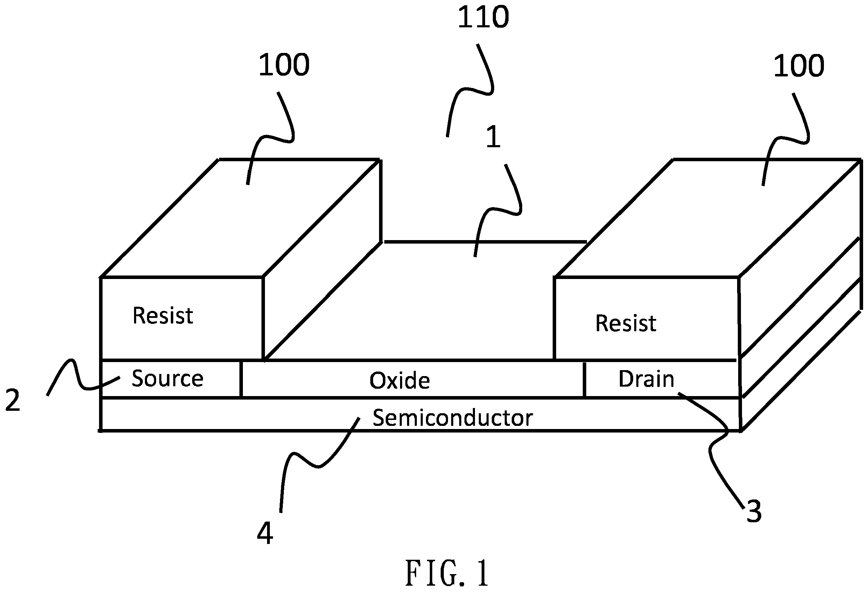

Fig. 1 is an exemplary illustration of basic device structure of a conventional semiconductor biosensor (See K. Koike, et al., Jpn. J. Appl. Phys., vol. 53, 05FF04 (2014).). An oxide film 1, a source electrode 2 and a drain electrode 3 are layout on a semiconductor substrate 4. Those electrodes are covered by a resist film 100 so as to be protected from a solution (e.g.: specimen such as blood, urine, sweat, and so forth) in which inspection target is dissociated. This conventional semiconductor biosensor is exposed into the solution during inspection.

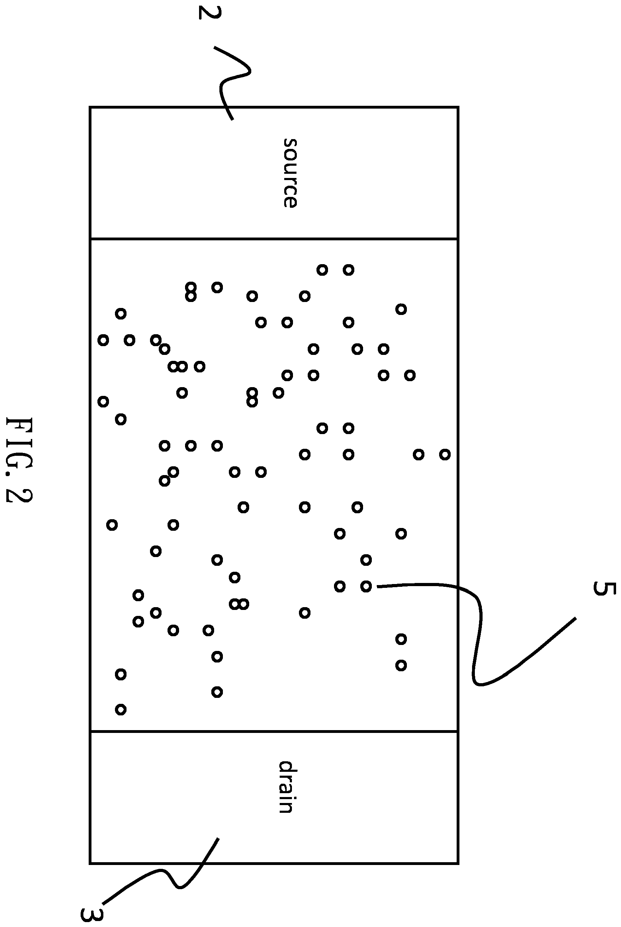

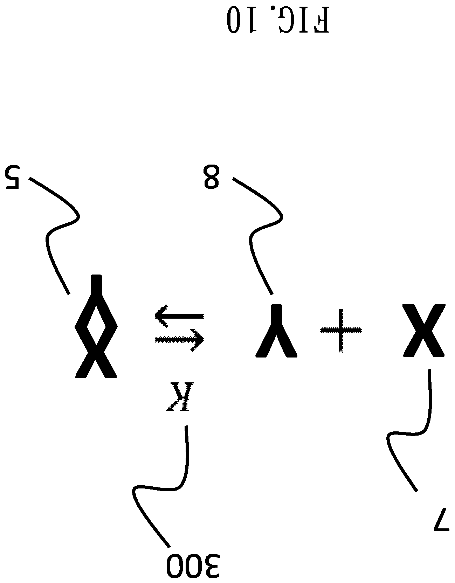

Referring to fig. 10, in the solution, a target 7 and a receptor 8 are attached to the surface of the oxide film 1 to produce a chemical reaction. The chemical reaction normally has a dissociation constant 300 (K) for determining the equilibrium state thereof. When the dissociation constant 300 is larger, the decoupling prevails in the chemical reaction. To the contrary, when the dissociation constant 300 is smaller, the coupling prevails in the chemical reaction, and thus a composite body 5 is made on the surface of the oxide film 1, as illustrated figs. 2, 3, and 4.

The composite body 5 has charge carried by the target 7. The charge will modulate surface electric field of the semiconductor substrate 4. It may capable of detecting whether the target 7 is contained in the solution by reading the change in electric current flowing between the source electrode 2 and the drain electrode 3.

There are many composite bodies 5 on the surface of the oxide film 1, as illustrated in fig. 2, as long as the dissociation constant 300 is small. If the dissociation constant 300 is larger, the density of the composite bodies 5 is decreased, as illustrated in figs. 3, and 4.

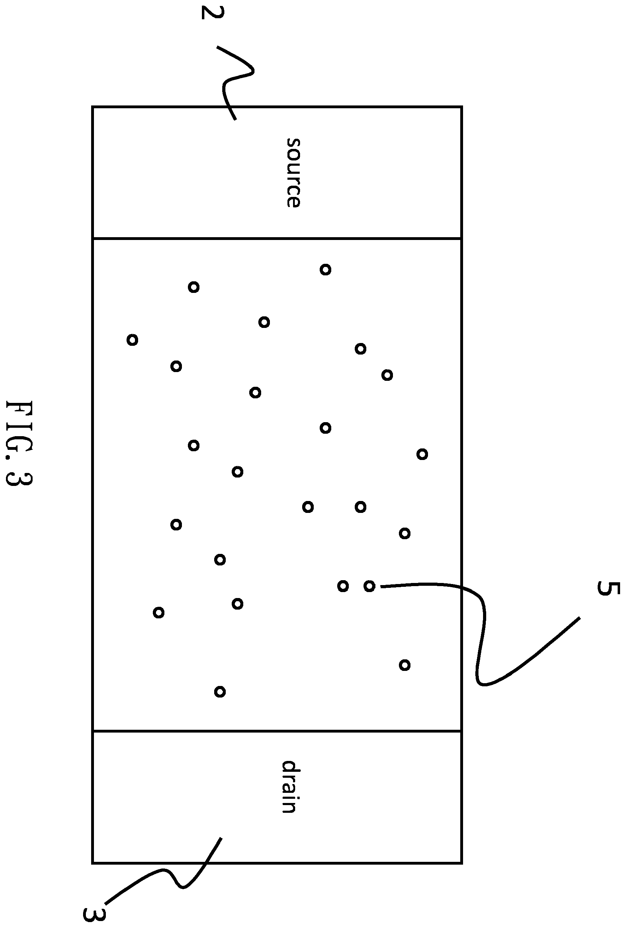

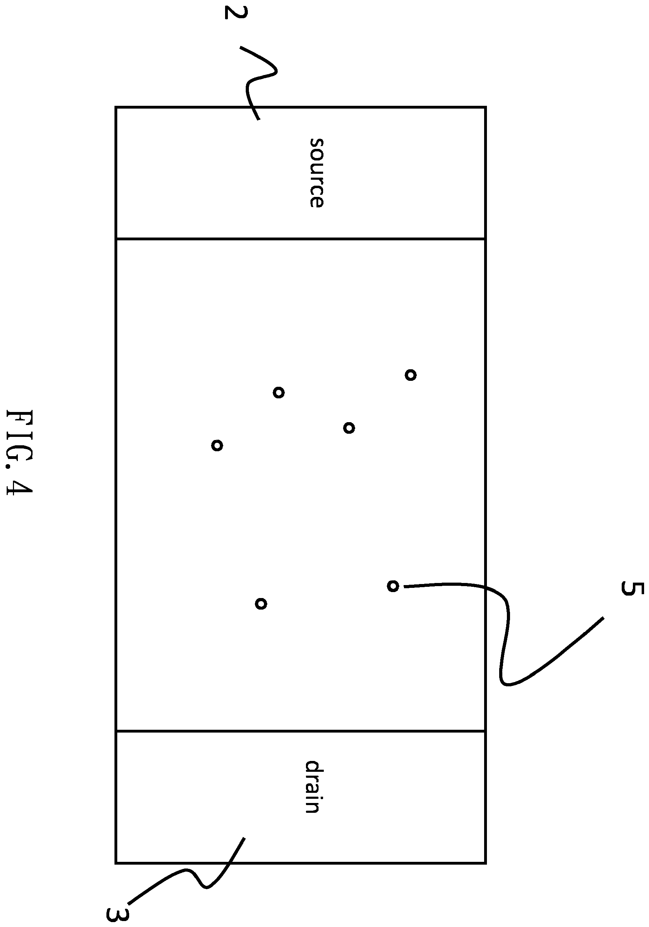

Besides, if the number of target 7 is more in solution, then more composite bodies 5 are attached to the surface of the oxide film 1, as illustrated in fig. 2. If the number of target 7 is less in solution, then, as illustrated in figs. 3, and 4, the number of composite bodies 5 attached to the surface of the oxide film 1 becomes less.

The dissociation constant 300 is sensitive to the density of target 7 in the solution and temperature.

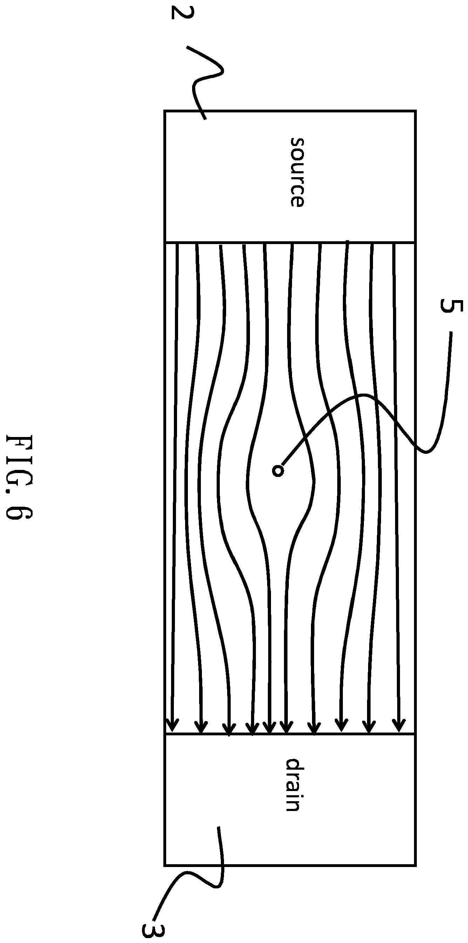

In figs. 3, and 4, those charges of the composite bodies 5 are sparse on the surface of the oxide film 1 and then work as point charges. In this regard, electrons flowing from the source electrode 2 to the drain electrode 3 can easily circumvent around the composite bodies 5, as shown in fig. 6. Thereby, if the electrons flowing from the source electrode 2 to the drain electrode 3 along a roundabout route, those composite bodies 5 exhibit no impact on the electric current characteristics of the conventional semiconductor biosensor.

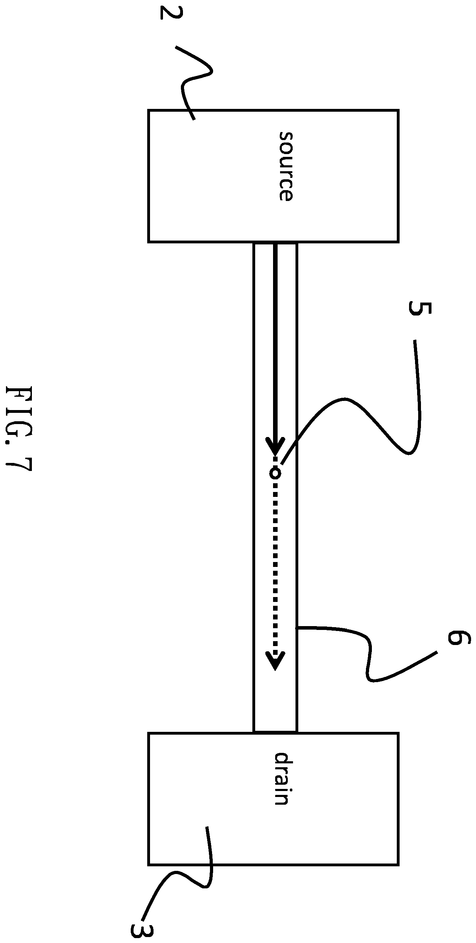

Therefore, referring to fig. 7, another conventional semiconductor biosensor replaces the semiconductor substrate 4 with wide gate width with a semiconductor conducting wire 6 with narrow gate width. Since electrons cannot circumvent around a composite body 5 while flowing through the semiconductor conducting wire 6, there is no roundabout route for the electrons flowing from the source electrode 2 to the drain electrode 3. Thus, the transport speed of those electrons is reduced in average and the electric current is suppressed. In theory, it may be possible to detect a sole target 7 as long as we can sense this change in the electric current.

In general, the change in electric current ($\Delta I_{ds}$) flowing on the semiconductor surface of transistors is sensed as change in threshold voltage shift ($\Delta V_t$). Depicting the trunsconductance $g_m$, the surface density of total charge carried by target 7 $Q_x$, the gate capacitance of transistor C, the surface density of receptors 8 [Y], the density of target 7 in solution [X], formula 1 is obtained with the cut-off to the background noise:

$$\frac{\Delta I_{ds}}{g_m} = \Delta V_t = \frac{Q_X}{C}[Y] \times \frac{[X]}{[X]+K} + \left(cut - off\right)$$

[Formula 1]

The cut-off is predetermined for veiling the noise not related to biosensors and must be much smaller than any noise attributable to the biosensors.

According to Formula 1, the limit of detection (LOD) is obtained as formula 2, where $I_{noise}$ is the absolute value of the electric current caused by the noise attributable to the biosensors. (See M. A. Reed, IEEE IEDM13, pp. 208-211 (2013).)

$$LOD = \frac{K}{\left(\dfrac{Q_X[Y]/C}{I_{noise}/g_m - \left(cut - off\right)}\right)}$$

[Formula 2]

According to Formula 2, it is found that LOD is made small as long as $I_{noise}$ becomes small and is equal to the cut-off.

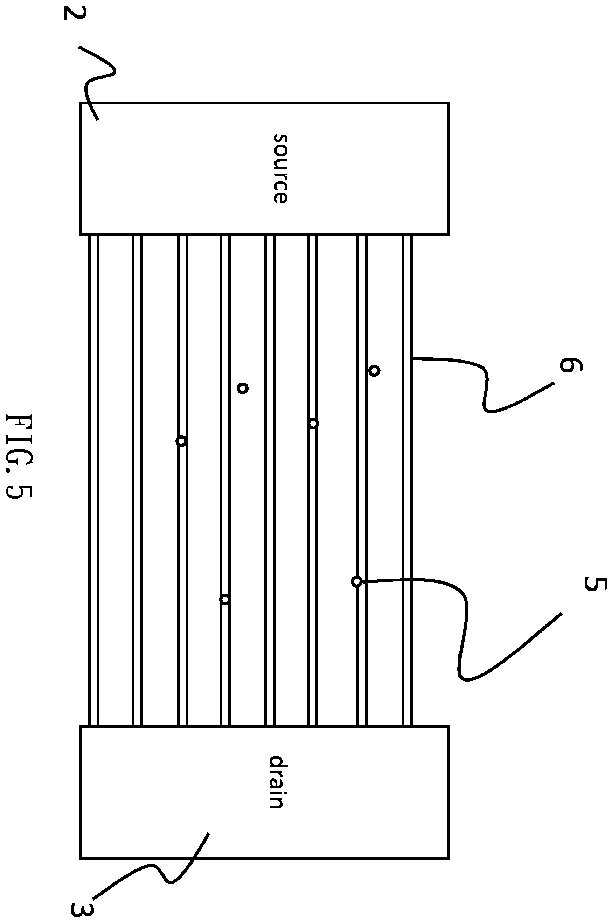

As mentioned above, $I_{noise}$ can be made small by using semiconductor conducting wires 6 to replace the semiconductor substrate 4. Specifically, referring to fig. 5, there are a plurality of conducting wires 6 in parallel between a common source 2 and a common drain 3. As example, there are three conducting wires 6, each of which can detect a sole composite body 5 on the surface of the oxide film 1. Then, it appears that the LOD is made small.

However, all of the conducting wires 6 are connected to the common drain 3, which indicates that the current signals from the conducting wires 6 are added. Once the signals are added up, it is impossible to distinguish signals from different conducting wires 6. Therefore, the LOD is not improved significantly.

SUMMARY OF THE INVENTION

An objective of the present disclosure is to provide a semiconductor biosensor and a control method thereof with improved limit of detection.

An embodiment of the semiconductor biosensors related to the present invention comprises a central reaction unit of a highly-precise very small inspection equipment, with the central reaction unit being able to be embedded into a semiconductor chip; and with the central reaction unit comprising: a plurality of semiconductor conducting wires; a common source, wherein one end of each conducting wire is connected to the common source; a plurality of non-volatile memory type transistors respectively connected to another end of each conducting wire; a plurality of sense-amplifiers respectively connected to the said non-volatile memory type transistors; a bit line controller analyzing signals sensed by the said sense-amplifiers and managing the operation of the said non-volatile memory type transistors; an oxide film wrapping or covering the said conducting wires, and a plurality of receptors fixed on a surface of the said oxide film.

A control method of the semiconductor biosensor comprises: initializing the semiconductor biosensor by sensing output signals from the plurality of conducting wires by the plurality of sense-amplifiers, and then testing for conducting wires having wire-errors, wherein conducting wires with anomalous high resistance or snapped conducting wires are regards as conducting wires having said wire-errors; data-thinning the semiconductor biosensor by selectively programming non-volatile type memory transistor connected to the conducting wires having said wire-errors; and exposing the semiconductor biosensor into a solution. In addition, the control method of the semiconductor biosensor, further comprising selectively tuning threshold voltages of the non-volatile memory type transistor connected to the conducting wires without said wire-errors after said data-thinning but before exposing the semiconductor biosensor into the solution.

In accordance to the above structure and method, it is made capable of producing a central reaction unit as a key device of the semiconductor biosensor to achieve highly-precise and very small inspection equipment.

Embodiments according to the present invention will be explained below with reference to the drawings. These embodiments are not intended to limit the present invention. The drawings schematically show practical devices and are not made to scale.

BRIEF DESCRIPTION OF THE DRAWINGS

The illustrative embodiments may best be described by reference to the accompanying drawings where:

Fig. 1 is a view illustrating a basic device structure of prior art biosensor.
Fig. 2 is a view illustrating a basic device structure of prior art biosensor.
Fig. 3 is a view illustrating a basic device structure of prior art biosensor.
Fig. 4 is a view illustrating a basic device structure of prior art biosensor.
Fig. 5 is a view illustrating a basic device structure of prior art biosensor;
Fig. 6 is a view illustrating that electrons flow around charge in prior art biosensor;
Fig. 7 is a view illustrating that electrons cannot flow around charge in prior art biosensor;

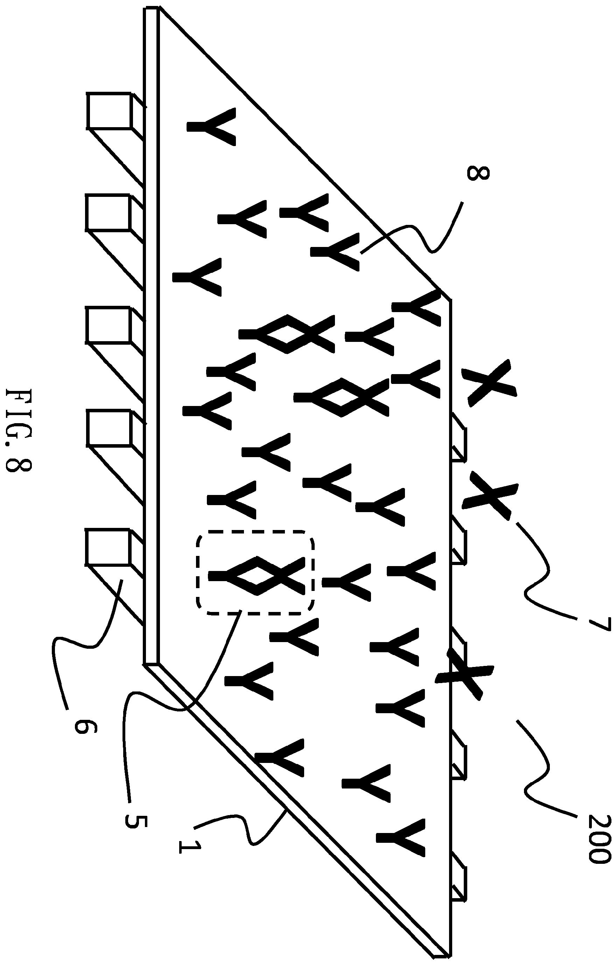

Fig. 8 is a view illustrating that receptors are attached on oxide surface and then targets moving in solution are caught by those receptors and immobilized;

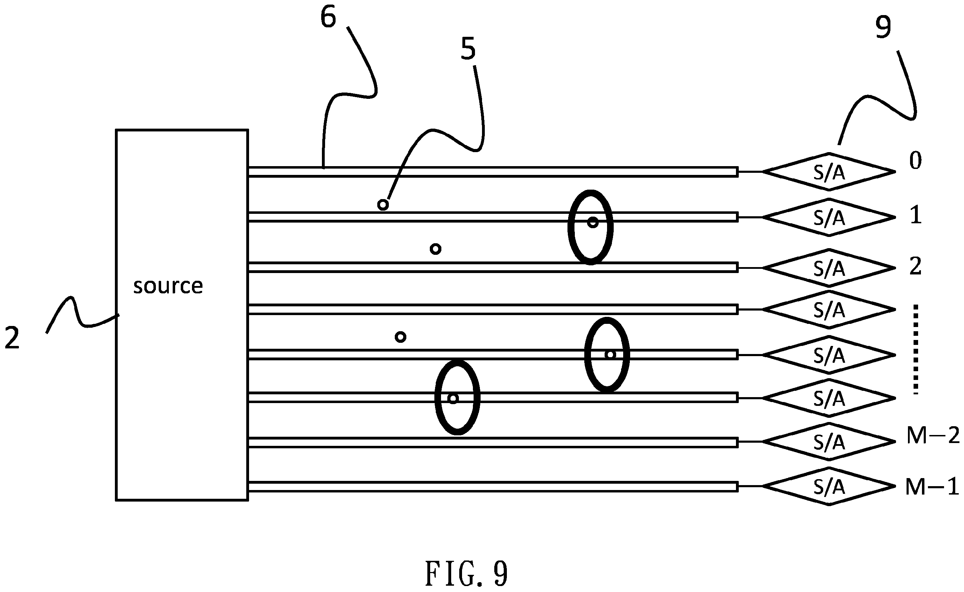

Fig. 9 is a view illustrating a basic component related to an embodiment of the present invention;
Fig. 10 is a view illustrating a reaction of receptors and targets, which is related to an embodiment of the present invention;

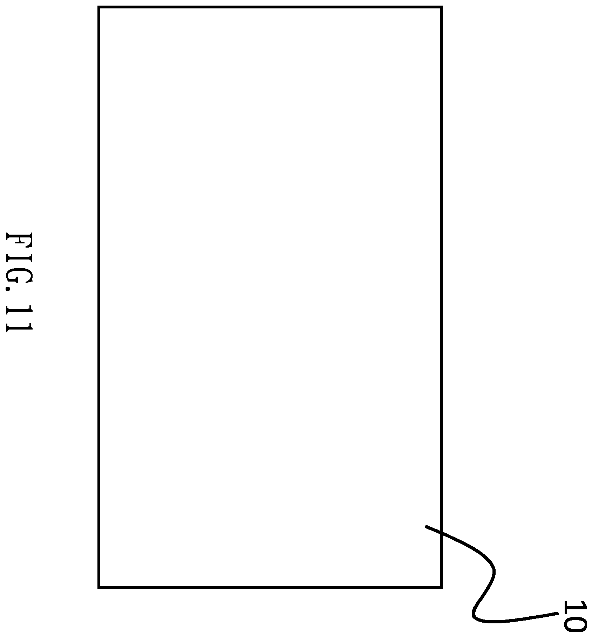

Fig. 11 is a view illustrating fabrication method of biosensor related to an embodiment of the present invention;

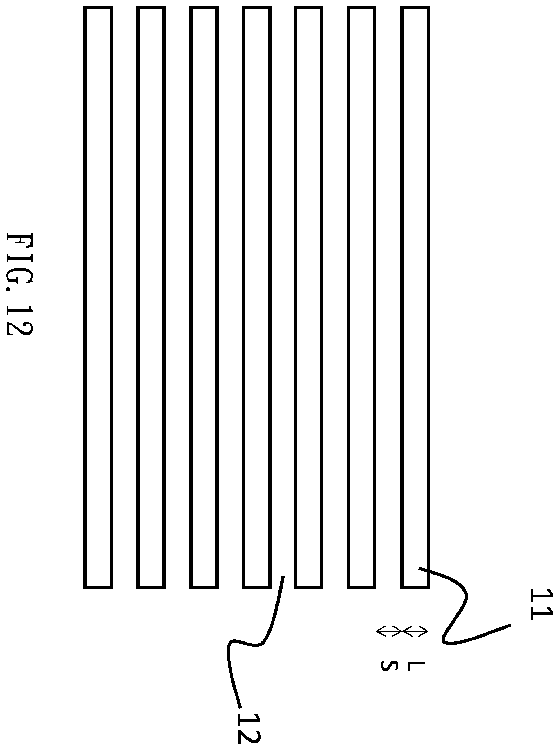

Fig. 12 is a view illustrating fabrication method of biosensor related to an embodiment of the present invention;

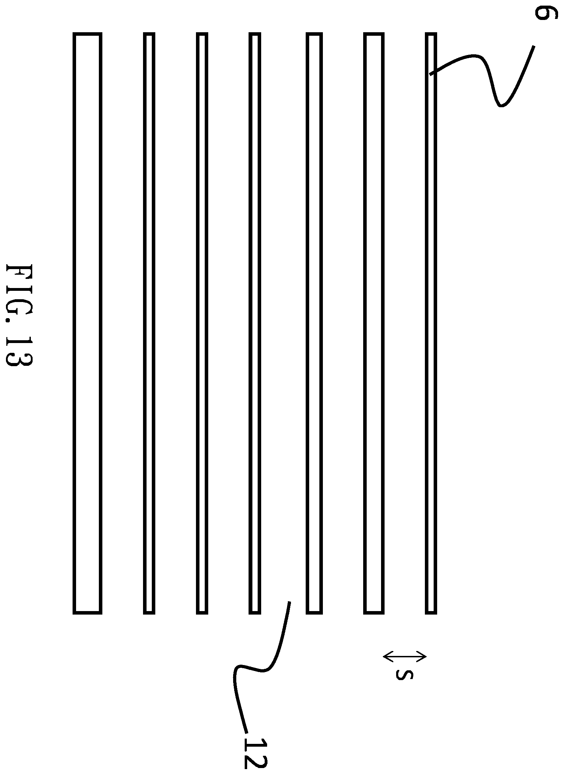

Fig. 13 is a view illustrating fabrication method of biosensor related to an embodiment of the present invention;

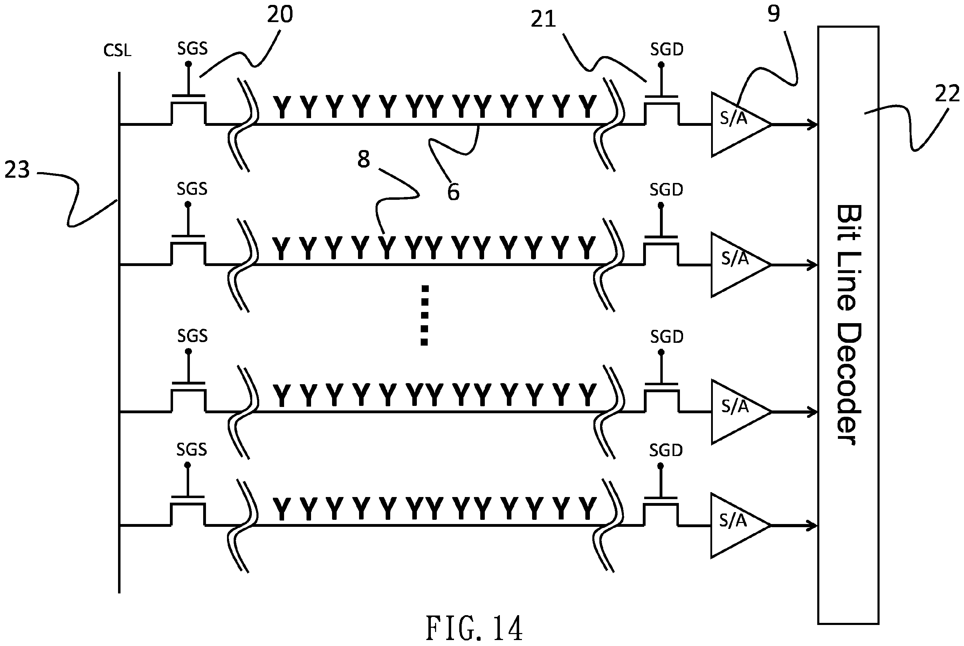

Fig. 14 is a view illustrating equivalent circuit related to an embodiment of the present invention;

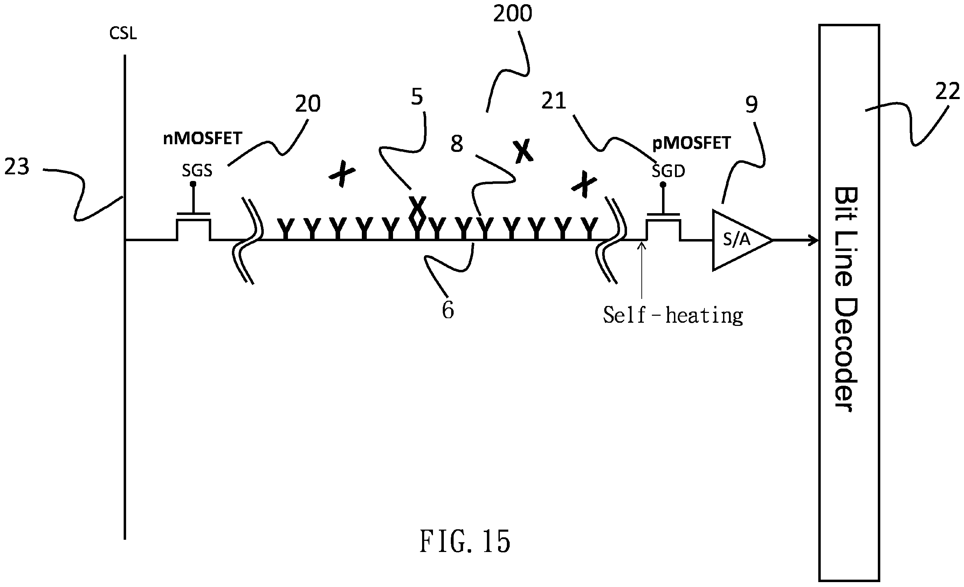

Fig. 15 is a view illustrating that those receptors catch targets in equivalent circuit of biosensor related to an embodiment of the present invention;

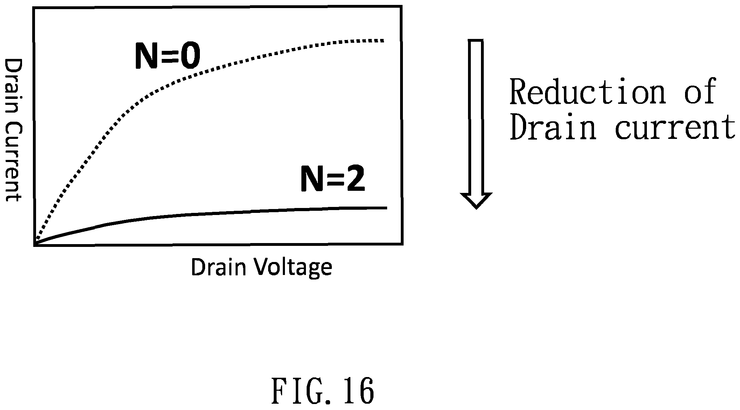

Fig. 16 is a view illustrating that signal electric current is modulated with regard to charge carried by those targets in biosensor related to an embodiment of the present invention;

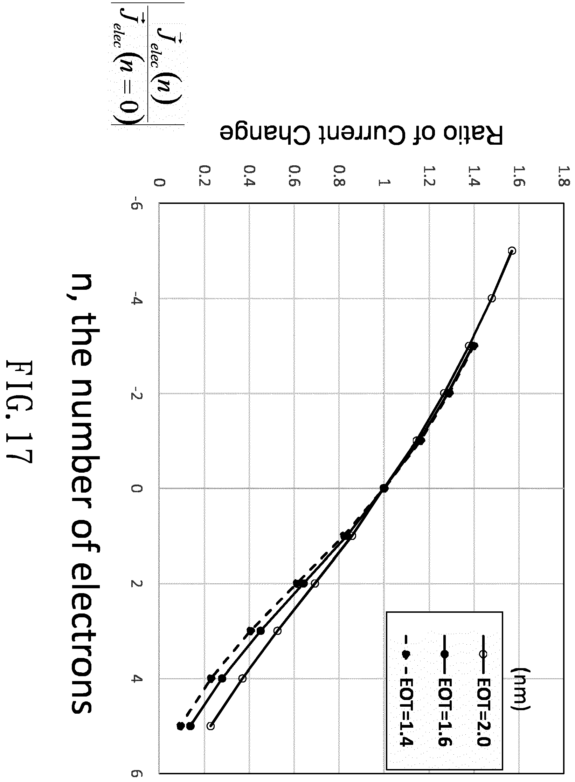

Fig. 17 is a view illustrating simulation result of operation of biosensor related to an embodiment of the present invention;

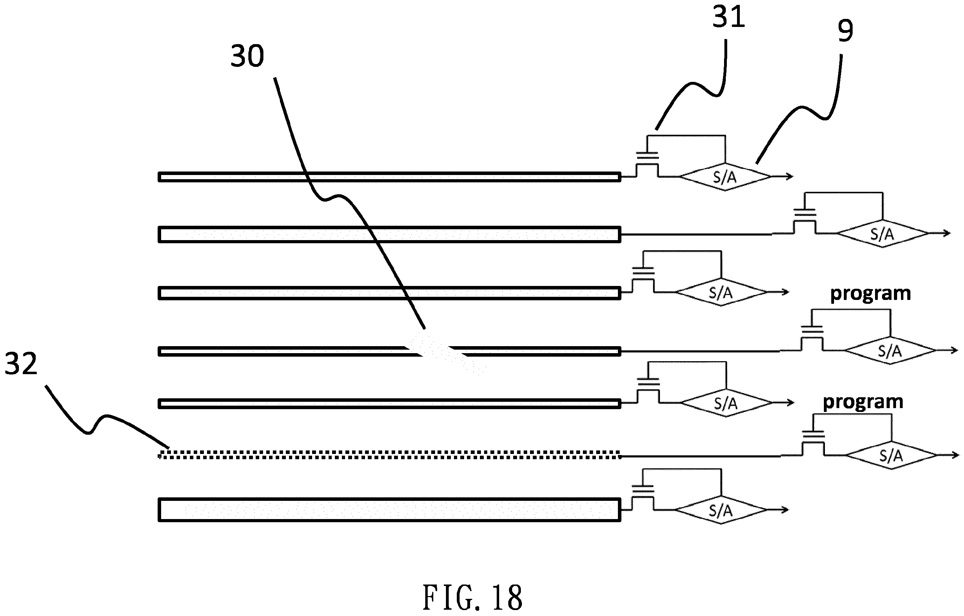

Fig. 18 is a view illustrating a method for correcting error modes of biosensor related to an embodiment of the present invention;

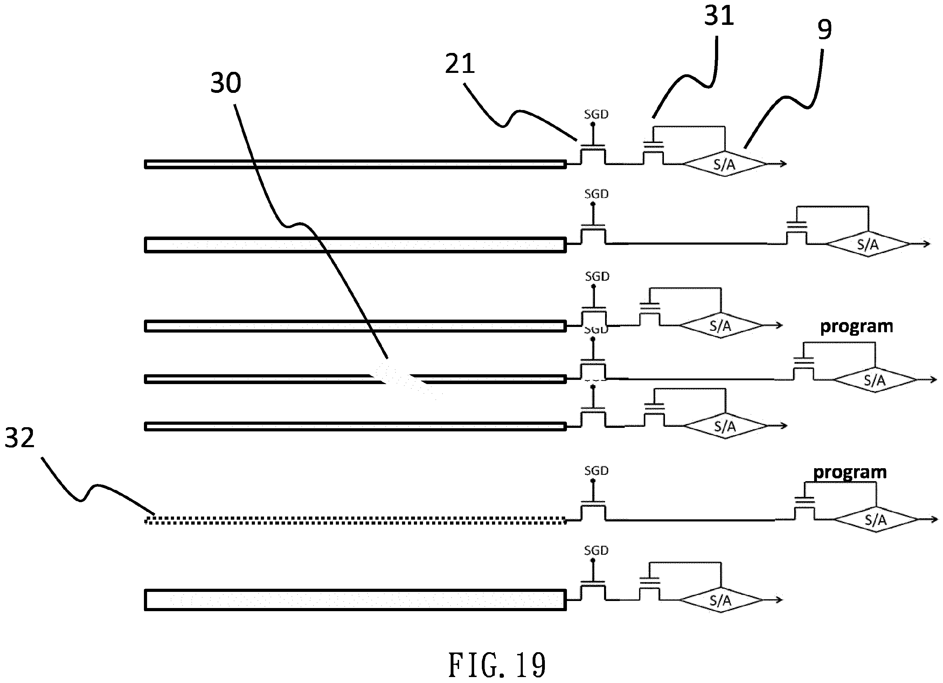

Fig. 19 is a view illustrating a method for correcting error modes of biosensor related to an embodiment of the present invention;

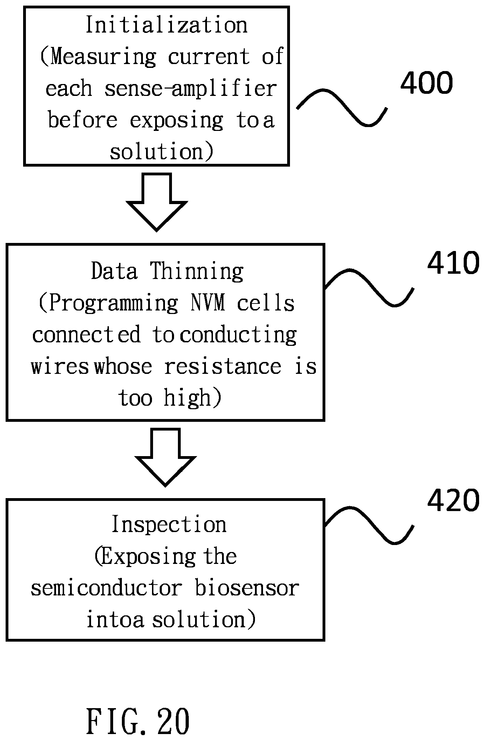

Fig. 20 is a view illustrating a method for correcting error modes of biosensor related to an embodiment of the present invention;

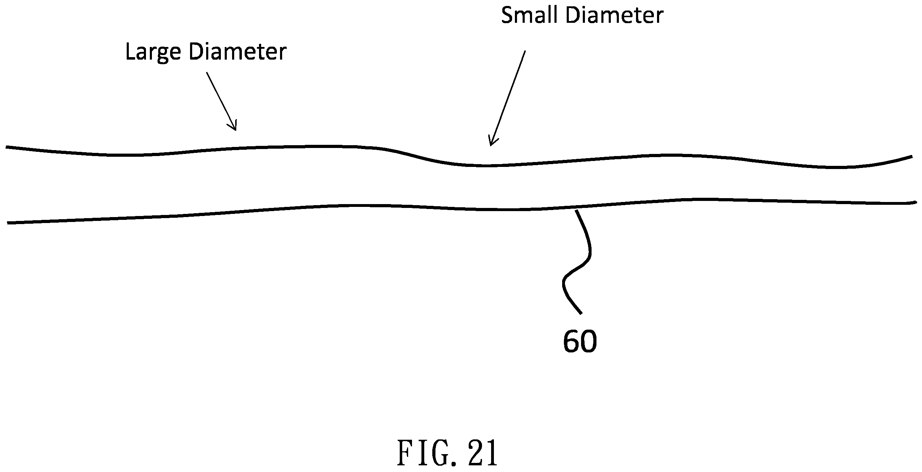

Fig. 21 is a view illustrating fluctuation of diameter of semiconductor conducting wires;

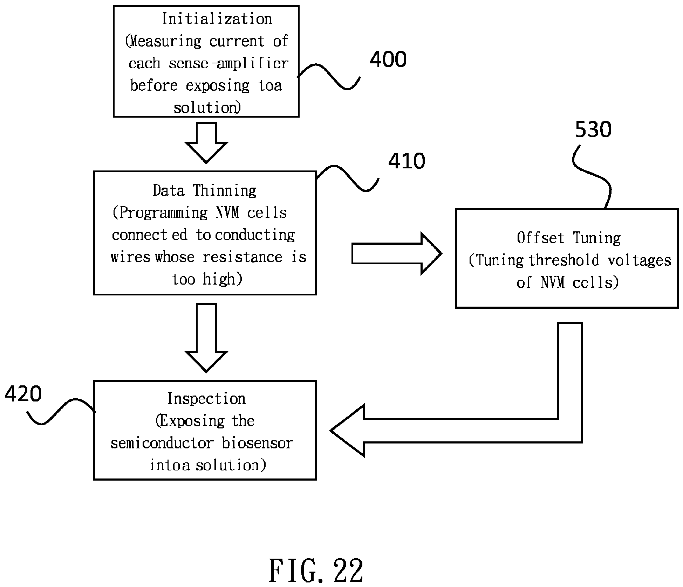

Fig. 22 is a view illustrating a method for correcting offset of biosensor related to an embodiment of the present invention.

DETAILED DESCRIPTION OF THE INVENTION

Referring to fig. 8, an embodiment of the semiconductor biosensor related to the present invention is constituted of a central reaction unit 200 comprising semiconductor conducting wires 6, an oxide film 1 and receptors 8, and the peripheral unit described below. The semiconductor conducting wires 6 can be thin conducting wires. Moreover, the semiconductor conducting wires 6 can be nanowires.

The central reaction unit 200 is fabricated on the semiconductor substrate. The central reaction unit 200 is exposed into a solution dissociating targets 7. The targets 7 having charge moves in the solution and then couples with receptors 8 attached to the surface of the oxide film 1 subject to the formula shown in fig. 10. The dissociation constant 300 determines the equilibrium state of the chemical reaction of the targets 7 and the receptors 8. When K is large, then receptors 8 and targets 7 are decoupled. When K is small, then the receptors 8 and the targets 7 are coupled to form immobilized composite bodies 5.

Referring to Fig. 9, the central reaction unit 200 further comprises a plurality of sense-amplifier 9 (S/A). One end of each conducting wire 6 is connected to a common source 2 and another end of each conducting wire 6 is connected to each sense-amplifier 9. The number of the sense-amplifier (M) is same with the number of the conducting wires 6 and the sense-amplifiers 9 are labeled from 0 to M-1, respectively.

In this figure, there are three conducting wires 6, each of which can detect a sole composite body 5 on the surface of the oxide film 1. The sense-amplifiers 9 corresponding to those conducting wires 6 can detect the reduction of current signal thanks to charges of those composite bodies 5. The difference between the embodiment of the semiconductor biosensor related to the present invention and the conventional semiconductor biosensor shown in fig. 5 is that the common drain 3 was replaced with a plurality of the sense-amplifiers 9 each of which is connected to one of the conducting wires 6 independently. By such arrangement, it is theoretically made possible to distinguish change in electric current from one of the conducting wires 6 attributable to a sole composite body 5, by not adding signals from every conducting wire 6 up.

Next, the method for distinguishing signal from noise is described. There are M conducting wires 6 and M sense-amplifiers 9. The M sense-amplifiers 9 and the common source 2 are connected at the opposite side of the M conducting wires 6 as shown in Fig. 9. It is supposed that m conducting wires 6 of M ones can detect sole composite body 5 on the surface of the oxide film 1 in a similar way. (In the example of fig. 9, m = 3.)

If one conducting wire 6 does not detect any sole composite body 5 on the surface of the oxide film 1, the electric current that flows from the common source 2 to the sense-amplifier 9 that is connected to said wire 6 is denoted as $I_0$. On the other hand, if one conducting wire 6 detects a sole composite body 5 on the surface of the oxide film 1, the electric current that flows from the common source 2 to the sense-amplifier 9 that is connected to said wire 6 is denoted as $I_1$. The electric current $I_1$ can be expressed as the sum of electric current $I_0$ and a difference $\Delta I$ between currents $I_0$ and $I_1$. Namely, $I_1 = I_0 + \Delta I$. In this regard, if the conventional semiconductor biosensor is used for inspection, the common drain 3 will receive an electric current from each conducting wire 6 that has a magnitude of $I_0 + (m/M) \times \Delta I$ in average. On the other hand, the sense-amplifier 9 of the biosensor in this embodiment, that is connected to the conducting wire 6 detecting the sole composite body 5 on the surface of the oxide film 1, is able to receive the electric current of $I_0 + \Delta I$. In other words, the sense-amplifier 9 in the embodiment is able to determine whether the conducting wire 6 detects the sole composite body 5 based on the electric current of $I_0 + \Delta I$. In contrast, the common drain 3 of the conventional biosensor can only determine whether a single conducting wire 6 detects the sole composite body 5 based on the electric current of $I_0 + (m/M) \times \Delta I$. The sense-amplifier 9 of the application receives an extra amount of current of $\Delta I \times (1 - m/M)$ in addition to the average current received by the conventional common drain 3. As such, the value of $(1 - m/M)$ can serve as a standard for evaluating the level of improvement to the Limit of Detection (LOD) in the embodiment.

The improving factor of LOD is given by Formula 3.

$$\varepsilon \cong 1 - \frac{m}{M}$$

[Formula 3]

Accidental current change on the conducting wires 6 is a noise, which misleading the conducting wires 6 without composite body 5 to be considered as having one. Assuming the number of the conducting wires 6 with noise is $\delta$. The noise is made ignorable as long as m is large enough compared with $\delta$. The total number of conducting wires 6 (M) should be made larger, in order to enlarge m while not degrading the limit of detection. Thereby, the improved LOD by the present invention, Formula 4, is obtained.

$$\text{Improved } LOD = (1 - \varepsilon) \times LOD = \frac{m}{M} \times LOD$$

[Formula 4]

In the example where the gate width of biosensor (i.e., the width of central reaction unit 200) is 2.4mm, the width of the conducting wire is 3nm in average, and the space between adjoining conducting wires 6 is 57 nm in average, there are 40,000 conducting wires 6. When the improving factor $\varepsilon$ (Formula 3) is 99.9%, m is 40. Indeed, there may be conducting wires 6 in which electric current is accidentally decreased. Namely, the possibility of the presence of the conducting wires 6 with noise is non-zero. However, the number of those conducting wires ($\delta$) may be less than 40. When the improving factor is 99%, m is 400 which may further larger than $\delta$. When the improving factor is 90%, m is 4000, which may much larger than $\delta$. Even for a 90% improving factor, the improving ratio (m/M) may be large enough.

The total number of the conducting wires 6 (M) is predetermined in the step of device design, which will be described below with an example of a fabrication method of the central reaction unit 200.

In fig. 11, there is a SOI (Silicon-On-Insulator) film 10 with the thickness being 20nm as an example. This SOI film 10 is cut out to line 11 and space 12 in the lithography process, as illustrated in fig. 12. The width of the line 11 (L) and the width of the space 12 (S) are 30nm. The lines 11 correspond to semiconductor wires. By this way, a plurality of semiconductor wires 11 with cross-section being (30nm, 30nm, 20nm) in average are made.

Next, an oxide film is compensated to the spaces 12, and then slim the semiconductor wires 11 by subsequent thermal processes (sliming process).

As a result, conducting wires 6 with diameter being 3nm in average and spaces 12 with width being 57nm in average are layout, as illustrated in fig. 13. Subsequently, CMP (Chemical and Mechanical Process) and oxidization are preceded. A thin oxide film 1 is formed after planarization to perform as gate oxide. Furthermore, receptors 8 are fixed on the surface of the oxide film 1, and then central reaction unit 200 is made in fig. 8.

Fig. 14 illustrates an equivalent circuit of the embodiment of the semiconductor biosensor related to the present invention. An end of conducting wire 6 is connected to a common source line (CSL) via a source select gate 20 (SGS).

The other end is connected to the sense-amplifier 9 via a drain select gate 21 (SGD). The signal from each sense-amplifier 9 is analyzed by a bit line decoder 22.

Fig. 15 is an illustration obtained by picking up a sole conducting wire 6 from the equivalent circuit and hiding the others in fig. 14. This is for paying attention to the operation of the conducting wire 6 related to the present invention. As an example, the source select gate 20 is an nMOSFET and the drain select gate 21 is a pMOSFET. In general, the four combinations of SGS 20 and SGD 21 are possible; for example, (nMOSFET and nMOSFET), (nMOSFET and pMOSFET), (pMOSFET and nMOSFET), and (pMOSFET and pMOSFET).

While both of the source select gate 20 and the drain select gate 21 are turned on, electron current is made flow from n-type diffusion layer of the source select gate 20 to the conducting wire 6 by applying drain voltage via the sense-amplifier 9. It is noted that the conducting wires 6 generally exhibit low thermal conductivity if the diameter is very small, so the heating dissipation is made difficult for the conducting wires 6, which leads to self-heating effect. Thus, in order to cool the conducting wires 6 down, it is preferable to dissipate the heat to p-type diffusion layer of the drain select gate 21 if the diameter is very small. Therefore, the drain select gate 21 can be a pMOSFET.

The electric current flowing through conducting wire 6 is made of electrons flowing therein. If the charge stored by composite bodies 5 is negative, the signal sensed by sense-amplifier 9 is reduced by the charge. Otherwise, the signal is increased by the charge.

Fig. 16 is an illustration of the drain current (current sensed by the sense-amplifiers 9) with no composite body 5 attached on the conducting wire 6 (N=0) and with the composite bodies 5 having two electrons attached on the conducting wire 6 (N=2). To sense the difference in current is to detect the existence of target 7.

The result of device simulation with a different amount of composite bodies 5 attached to the conducting wire 6 is shown in fig. 17. The ratio of the current to that with no composite body 5; which is neutral (N=0), is plotted with respect to the number of electrons (n) stored in the composite body 5. As the number of electrons increasing, the ratio of the current is reduce to about half at n=3, or about 20% at n=4. It is able to detect the current change with this level of reduction with standard sense-amplifier.

The EOT is the Equivalent Oxide Thickness of some dielectric film between the target 7 and the conducting wire 6, to which the thickness of the dielectric film is converted. The sensitivity is improved as EOT is decreased. It is preferable that EOT is less than 2nm from this simulation result.

As illustrated in fig. 13, the production tolerance is not negligible in actually fabricated line-and-space structures. The resistivity of the conducting wire 6 is increased as the diameter of the conducting wire 6 becomes smaller; and is decreased as it becomes larger. This fluctuation of the diameter of the conducting wire 6 induces the noise contaminated into signals sensed by the sense-amplifiers 9.

Referring to fig. 18 and 19, the resistance of conducting wires with diameter being too small 32 is high enough to make the signal undistinguishable from noise. On the other hand, there may also be snapped conducting wire 30. The snapped conducting wire 30 cannot conduct current, and thus the signal from which is also undistinguishable from noise.

Figs. 18 and 19 illustrate a control method of the semiconductor biosensor related to the present invention for dealing with the production tolerance. In fig. 18, the drain select gate 21 is replaced with non-volatile memory type transistor 31. On the other hand, in fig. 19, non-volatile memory type transistor 31 is put between the drain select gate 21 and the sense-amplifier 9.

Firstly, as illustrated in fig. 20, the electric current is sensed by the sense-amplifier 9 while the central reaction 200 unit is exposed into a solution without target 7 or not exposed into any solution. This is the step of Initialization. Conducting wires 6 with no sensible current are regarded as conducting wires with diameter being too small 32 or as snapped conducting wires 30. Then, non-volatile memory type transistor 31 related to those conducting wires 6 is programmed. Since the programmed non-volatile memory type transistors 31 are turned off, the data of those conducting wires 6 are not transferred to sense-amplifier 90. This is the step of Data Thinning. After excluding the conducting wires with diameter being too small 32 and snapped conducting wires 30, the left-behind conducting wires 6 are utilized for testing by sensing the electric current via the sense-amplifiers 9. This is the step of Inspection.

In general, the operation of transistor composed of conducting wire 6 is more influenced by surface states than the conventional MOSFET is. It is because the surface to the volume is larger in conducting wire 6 than in a substrate constituting the conventional MOSFET. Thereby, more noise is contaminated to the signal through conducting wire 6 than the signal on the surface of the substrate. The cut-off shown in Formula 2 is determined with respect to the maximum amplitude of the noise.

The amplitude of noise though conducting wire 6 is sensitive to the diameter of conducting wire 6. As long as the cut-off is adequate, the amplitude of the noise is less than the limitation of control. Of course, the conducting wires with diameter being too small 32 or with anomalously high resistance may induce noise with amplitude out of the limitation, so the conducting wires with diameter being too small 32 should be excluded.

It is necessary to take the fluctuation (increase and the decrease) in the amplitude of the noise into consideration for adequately determining the cut-off.

In addition, as illustrated in fig. 21, there is fluctuation in diameter even within a sole conducting wire 60, which is a characteristic of the conducting wire 6. It is also necessary to take the fluctuation in the diameter into consideration for adequately determining the cut-off.

Since it is impossible to grasp fluctuation in the amplitude of the noise or the fluctuation in the diameter when designing biosensor, it is necessary to tune the cut-off to suppress the impact of those fluctuations.

In concrete, the system with non-volatile memory type transistor 31, constituting an exemplary embodiment related to the present invention and illustrated in figs. 18 and 19, is capable of adequately determining the cut-off.

In another embodiment of the control method of the semiconductor biosensor related to the present invention, as illustrated in fig. 22, the step of Offset Tuning 530 is appended next to the step of Data Thinning 410 in the flow chart illustrated in fig. 20. In this step, the resistance of non-volatile memory type transistor 31 is tuned by arranging threshold voltage of the non-volatile memory type transistor 31, and thereby, suppressing the impact of fluctuation in the amplitude of the noise or the diameter

The method to arrange threshold voltage of non-volatile memory type transistor 31 is well-known as verify programming, in which program-erase is repeated with small step (short pulse). (See T. Tanaka, et al., 1990 Symposium on VLSI circuits, pp. 105-106 (1990).)

By the embodiments of present invention, the limit of detection of biosensor is substantially improved, which result in the significant enhancement of performance of semiconductor biosensor and the drastic price reduction of medical healthcare chip. This enables early detection of disease, which has been impossible with the conventional biosensors, and then substantially reduces medical cost.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

**Claims**

1. A semiconductor biosensor, comprising:

    a central reaction unit (200) capable of being embedded into a semiconductor chip; and with the central reaction unit (200) comprising:

        a common source electrode (2);
        a bit line controller (22);
        an oxide film (1), and
        a plurality of receptors (8) adapted for coupling with at least one charged target (7) fixed on a surface of the said oxide film (1);

    wherein the central reaction unit (200) comprises is **characterized in**

        a plurality of conducting wires (6), wherein one end of each conducting wire (6) is connected to the common source electrode (2);
        a plurality of non-volatile memory type transistors (31) respectively connected to another end of each conducting wire (6);
        a plurality of sense-amplifiers (9) respectively connected to each of said non-volatile memory type transistors (31), with the bit line controller (22) analyzing signals sensed by each of said sense-amplifiers (9) and managing the operation of the said non-volatile memory type transistors (31), and with the oxide film (1) wrapping or covering the said conducting wires (6).

2. The semiconductor biosensor as claimed in claim 1, **characterized in that** the central reaction unit (200) further comprises a plurality of drain select gate transistors respectively connected between the said conducting wires (6) and the said non-volatile memory type transistors (31).

3. A control method for the semiconductor biosensor of claim 1, **characterized in** comprising:

    initializing the semiconductor biosensor by sensing output signals from the plurality of conducting wires (6) by the plurality of sense-amplifiers (9), and then testing for conducting wires (6) having wire-errors, wherein conducting wires with anomalously high resistance (32) or snapped conducting wires (30) are regards as conducting wires (6) having said wire-errors;

reducing data transferred inside the semiconductor biosensor by selectively programming non-volatile type memory transistor (31) connected to the conducting wires (6) having said wire-errors; and

exposing the semiconductor biosensor into a solution.

**4.** The control method of the semiconductor biosensor as claimed in claim 3, further comprising selectively tuning threshold voltages of the non-volatile memory type transistor (31) connected to the conducting wires (6) without said wire-errors after said reducing data transferred inside the semiconductor and before exposing the semiconductor biosensor into the solution.

**5.** The control method for the semiconductor biosensor of claim 3, **characterized in that** testing for conducting wires (6) having said wire-errors comprises:

locating conducting wires (6) with no sensible electric current according to the output signals sensed by the plurality of sense-amplifiers (9); and

regarding the conducting wires (6) with no sensible current as conducting wires with anomalously high resistance (32) or snapped conducting wires (30).

**Patentansprüche**

**1.** Halbleiterbiosensor, der Folgendes umfasst:

eine zentrale Reaktionseinheit (200), die in einen Halbleiterchip eingebettet werden kann; und wobei die zentrale Reaktionseinheit (200) umfasst:

eine gemeinsame Source-Elektrode (2);
eine Bitleitungssteuereinheit (22);
einen Oxidfilm (1), und
mehrere Rezeptoren (8), die ausgelegt sind, sich mit wenigstens einem geladenen Ziel (7) zu koppeln und auf einer Oberfläche des Oxidfilms (1) befestigt sind;

wobei die zentrale Reaktionseinheit (200) **dadurch gekennzeichnet ist, dass** sie umfasst:

mehrere Leitungsdrähte (6), wobei ein Ende jedes Leitungsdrahts (6) mit der gemeinsamen Source-Elektrode (2) verbunden ist;
mehrere Transistoren (31) vom nichtflüchtigen Speichertyp, die jeweils mit einem weiteren Ende jedes Leitungsdrahts (6) verbunden sind;
mehrere Leseverstärker (9), die jeweils mit jedem der Transistoren (31) vom nichtflüchtigen Speichertyp verbunden sind, wobei die Bitleitungssteuereinheit (22) Signale analysiert, die durch jeden der Leseverstärker (9) erfasst werden, und den Betrieb der Transistoren (31) vom nichtflüchtigen Speichertyp managt und wobei der Oxidfilm (1) die Leitungsdrähte (6) umhüllt oder bedeckt.

**2.** Halbleiterbiosensor nach Anspruch 1, **dadurch gekennzeichnet, dass** die zentrale Reaktionseinheit (200) ferner mehrere Drain-Auswahl-Gate-Transistoren umfasst, die jeweils zwischen den Leitungsdrähten (6) und den Transistoren (31) vom nichtflüchtigen Speichertyp verbunden sind.

**3.** Steuerfahren für den Halbleiterbiosensor nach Anspruch 1, **gekennzeichnet durch**:

Initialisieren des Halbleiterbiosensors **durch** Erfassen von Ausgangssignalen aus den mehreren Leitungsdrähten (6) **durch** die mehreren Leseverstärker (9) und dann Überprüfen auf Leitungsdrähte (6), die Drahtfehler aufweisen, wobei Leitungsdrähte mit anomal hohem Widerstand (32) oder gebrochene Leitungsdrähte (30) als Leitungsdrähte (6) betrachtet werden, die die Drahtfehler aufweisen;
Reduzieren von Daten, die innerhalb des Halbleiterbiosensors übertragen werden, **durch** selektives Programmieren des Transistors (31) vom nichtflüchtigen Speichertyp, der mit den Leitungsdrähten (6), die die Drahtfehler aufweisen, verbunden ist; und
Aussetzen des Halbleiterbiosensors in eine Lösung.

**4.** Steuerverfahren des Halbleiterbiosensors nach Anspruch 3, das ferner selektives Abstimmen von Schwellenspannungen des Transistors (31) vom nichtflüchtigen Speichertyp, der mit den Leitungsdrähten (6) ohne die Drahtfehler verbunden ist nach dem Reduzieren von Daten, die innerhalb des Halbleiters übertragen werden, und vor dem Aussetzen des Halbleiterbiosensors in die Lösung umfasst.

**5.** Steuerverfahren für den Halbleiterbiosensor nach Anspruch 3, **dadurch gekennzeichnet, dass** das Überprüfen auf Leitungsdrähte (6), die die Drahtfehler aufweisen, Folgendes umfasst:

Lokalisieren von Leitungsdrähten (6) mit keinem erfassbaren elektrischen Strom gemäß den Eingangssignalen, die durch die mehreren Leseverstärker (9) erfasst werden; und
Betrachten der Leitungsdrähte (6) mit keinem erfassbaren Strom als Leitungsdrähte mit anomal hohem Widerstand (32) oder gebrochene Leitungsdrähte (30).

**Revendications**

**1.** Biocapteur semi-conducteur, comprenant :

une unité de réaction centrale (200) capable d'être intégrée dans une puce semi-conductrice ; et l'unité de réaction centrale (200) comprenant :

une électrode de source commune (2) ;
un contrôleur de ligne de bit (22) ;
un film d'oxyde (1), et
une pluralité de récepteurs (8) conçus pour être couplés à au moins une cible chargée (7) et fixés sur une surface dudit film d'oxyde (1) ;

dans lequel l'unité de réaction centrale (200) comprend :

une pluralité de fils conducteurs (6), dans lequel une extrémité de chaque fil conducteur (6) est connectée à l'électrode de source commune (2) ;
une pluralité de transistors de type de mémoire non volatile (31) respectivement connectés à une autre extrémité de chaque fil conducteur (6) ;
une pluralité d'amplificateurs de lecture (9) respectivement connectés à chacun desdits transistors de type de mémoire non volatile (31), le contrôleur de ligne de bit (22) analysant les signaux détectés par chacun desdits amplificateurs de lecture (9) et gérant le fonctionnement desdits transistors de type de mémoire non volatile (31), et le film d'oxyde (1) s'enroulant autour desdits fils conducteurs (6) ou recouvrant ceux-ci.

**2.** Biocapteur semi-conducteur selon la revendication 1, **caractérisé en ce que** l'unité de réaction centrale (200) comprend en outre une pluralité de transistors à grille de sélection de drain respectivement connectés entre lesdits fils conducteurs (6) et lesdits transistors de type de mémoire non volatile (31).

**3.** Procédé de commande pour le biocapteur semi-conducteur selon la revendication 1, **caractérisé en ce qu'**il comprend :

l'initialisation du biocapteur semi-conducteur en détectant les signaux de sortie de la pluralité de fils conducteurs (6) par la pluralité d'amplificateurs de lecture (9), et en testant ensuite les fils conducteurs (6) présentant des erreurs de fil, dans lequel les fils conducteurs avec une résistance anormalement élevée (32) ou les fils conducteurs cassés (30) sont considérés comme des fils conducteurs (6) présentant lesdites erreurs de fil ;
la réduction des données transférées à l'intérieur du biocapteur semi-conducteur en programmant de manière sélective le transistor de type de mémoire non volatile (31) connecté aux fils conducteurs (6) présentant lesdites erreurs de fil ; et
l'exposition du biocapteur semi-conducteur dans une solution.

**4.** Procédé de commande du biocapteur semi-conducteur selon la revendication 3, comprenant en outre l'accord de manière sélective des tensions de seuil du transistor de type de mémoire non volatile (31) connecté aux fils conducteurs (6) sans lesdites erreurs de fil après ladite réduction des données transférées à l'intérieur du semi-conducteur et avant l'exposition du biocapteur semi-conducteur dans la solution.

**5.** Procédé de commande pour le biocapteur semi-conducteur selon la revendication 3, **caractérisé en ce que** le test des fils conducteurs (6) présentant lesdites erreurs de fil comprend :

la localisation des fils conducteurs (6) sans courant électrique sensible conformément aux signaux de sortie détectés par la pluralité d'amplificateurs de lecture (9) ; et
la considération des fils conducteurs (6) sans courant sensible en tant que fils conducteurs avec une résistance anormalement élevée (32) ou fils conducteurs cassés (30).

FIG. 1

EP 2 982 981 B1

FIG. 2

EP 2 982 981 B1

5

2

source

drain

3

FIG. 3

EP 2 982 981 B1

FIG. 4

FIG. 5

source

drain

2

6

5

3

EP 2 982 981 B1

FIG. 6

FIG. 7

FIG. 8

EP 2 982 981 B1

FIG. 9

EP 2 982 981 B1

FIG. 10

10

FIG. 11

FIG. 12

FIG. 13

6

12

S

EP 2 982 981 B1

FIG. 14

EP 2 982 981 B1

CSL

23

**nMOSFET**
SGS

20

5

200

8

21

**pMOSFET**
SGD

9

22

Bit Line Decoder

6

Self-heating

S/A

FIG. 15

EP 2 982 981 B1

FIG. 16

FIG. 17

EP 2 982 981 B1

FIG. 18

FIG. 19

EP 2 982 981 B1

Initialization
(Measuring current of
each sense-amplifier
before exposing to a
solution)

400

Data Thinning
(Programming NVM cells
connected to conducting
wires whose resistance is
too high)

410

Inspection
(Exposing the
semiconductor biosensor
into a solution)

420

FIG. 20

Large Diameter

Small Diameter

60

FIG. 21

```
┌─────────────────────┐
│    Initialization    │
│ (Measuring current of│      400
│ each sense-amplifier │
│ before exposing toa  │
│      solution)       │
└─────────────────────┘
          │
          ▼
┌─────────────────────┐                           530
│    Data Thinning     │      410
│ (Programming NVM cells│                    ┌─────────────────────┐
│ connect ed to conducting│   ⟹              │    Offset Tuning     │
│ wires whose resistance is│                 │(Tuning threshold voltages│
│      too high)       │                     │    of NVM cells)     │
└─────────────────────┘                     └─────────────────────┘
          │                                            │
          ▼                                            │
   420  ┌─────────────────────┐                        │
        │     Inspection      │  ⟸────────────────────┘
        │ (Exposing the       │
        │ semiconductor biosensor│
        │   intoa solution)   │
        └─────────────────────┘
```

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8697565 B2

### Non-patent literature cited in the description

- **K. KOIKE et al.** *Jpn. J. Appl. Phys.,* 2014, vol. 53, 05FF04
- **M. A. REED.** *IEEE IEDM13,* 2013, 208-211
- **T. TANAKA et al.** *Symposium on VLSI circuits,* 1990, 105-106